# EUROPEAN PATENT SPECIFICATION

(11) **EP 3 922 713 B1**
(45) Date of publication and mention of the grant of the patent: **07.05.2025**
(21) Application number: 20753257.3
(22) Date of filing: 03.02.2020
(51) Int. Cl.: C12N 1/12, C12M 1/00

(54) **HAPTOPHYTE CULTURING METHOD AND HAPTOPHYTE CULTURING DEVICE**
ZÜCHTUNGSVERFAHREN FÜR HAPTOPHYTEN UND ZÜCHTUNGSVORRICHTUNG FÜR HAPTOPHYTEN
DISPOSITIF DE CULTURE CELLULAIRE ET DISPOSITIF DE CULTURE D'HAPTOPHYTE

(30) Priority: 04.02.2019 JP 2019017684
(43) Date of publication of application: 15.12.2021
(73) Proprietor: Hamamatsu Photonics K.K., Hamamatsu-shi, Shizuoka 435-8558 (JP)
(72) Inventor: MATSUNAGA Shigeru, Hamamatsu-shi, Shizuoka 435-8558 (JP); ITOH Hiroyasu, Hamamatsu-shi, Shizuoka 435-8558 (JP)
(74) Representative: Hoffmann Eitle
(86) International application number: PCT/JP2020/003963
(87) International publication number: WO 2020/162407

(56) References cited:
- WO-A1-2005/102031
- JP-A- 2002 315 569
- JP-A- 2011 030 463
- JP-A- 2015 510 768
- DE MOOIJ TIM ET AL: "Impact of light color on photobioreactor productivity", ALGAL RESEARCH, vol. 15, 1 April 2016 (2016-04-01), NL, pages 32 - 42, XP055959793, ISSN: 2211-9264, Retrieved from the Internet <URL:http://dx.doi.org/10.1016/j.algal.2016.01.015> DOI: 10.1016/j.algal.2016.01.015
- KIM SO HEE ET AL: "Lipid and unsaturated fatty acid productions from three microalgae using nitrate and light-emitting diodes with complementary LED wavelength in a two-phase culture system", BIOPROCESS AND BIOSYSTEMS ENGINEERING, SPRINGER, DE, vol. 42, no. 9, 20 May 2019 (2019-05-20), pages 1517 - 1526, XP036850284, ISSN: 1615-7591, [retrieved on 20190520], DOI: 10.1007/S00449-019-02149-Y
- ISHIKAWA, T. ; ISOWA, K.: "Cultivation of Microalgae for Live Food under White Light-emitting Diodes (LEDs)", JOURNAL OF FISHERIES TECHNOLOGY, vol. 4, no. 2, 30 November 2011 (2011-11-30), JP, pages 51 - 55, XP009529844, ISSN: 1883-2253
- YAGO, TAKAHIDE ET AL.: "Effects of difference in wavelength of intermittent irradiation light on growth and fatty acid composition of the haptophyte Isochrysis galbana", LECTURE ABSTRACTS OF ACADEMIC RESEARCH CONFERENCE OF JAPANESE-FRENCH OCEANOGRAPHIC SOCIETY, 30 November 2007 (2007-11-30), JP, pages 4, XP009529843
- RA C. H. ET AL.: "Effects of light-emitting diode (LED) with a mixture of wavelengths on the growth and lipid content of microalgae", BIOPROCESS AND BIOSYSTEMS ENGINEERING, vol. 41, 2018, pages 457 - 465, XP036457223, DOI: 10.1007/s00449-017-1880-1

## Description

### Technical Field

The present disclosure relates to a haptophyte culturing method and a haptophyte culturing device.

### Background Art

Patent Document 1 discloses an algae culturing method for promoting growth by irradiating algae with artificial light. In this algae culturing method, an irradiation cycle including a red light irradiation procedure for irradiating the algae with red light and a blue light irradiation procedure for irradiating the algae with blue light is performed at least twice within a certain period of time.

### Citation List

### Patent Document

Patent Document 1: Japanese Unexamined Patent Publication No. 2015-128448

### Summary of Invention

### Technical Problem

It is confirmed that haptophytes grow and yet decrease in a short period of time when the haptophytes are cultured by being irradiated with the red or blue light pertaining to the algae culturing method described in Patent Document 1. An object of the present disclosure is to provide a culturing method and a culturing device enabling to extend the life of the haptophytes.

### Solution to Problem

The present inventor has found that the period from the initiation of culturing by light irradiation to a decrease in population is longer when haptophytes are irradiated with both yellow light and non-yellow visible light than when the haptophytes are irradiated with the non-yellow visible light alone.

Based on the above findings, the haptophyte culturing method according to the present disclosure includes irradiating culture liquid containing the haptophytes with both yellow light and non-yellow visible light. According to this haptophyte culturing method, the haptophytes grow by receiving the yellow light and the non-yellow visible light. As a result, by this culturing method, the life of the haptophytes can be extended as compared with the case of irradiation with the non-yellow visible light alone.

In one embodiment, the haptophytes may be Pavlova lutheri. In this case, the life of Pavlova lutheri can be extended.

The haptophyte culturing device according to another aspect of the present disclosure includes: a culture tank storing culture liquid containing the haptophytes; a first light source irradiating the culture liquid in the culture tank with yellow light; and a second light source irradiating the culture liquid in the culture tank with non-yellow visible light. According to this haptophyte culturing device, haptophytes are cultured in the culture liquid of the culture tank. The haptophytes grow by receiving both the yellow light emitted from the first light source and the non-yellow visible light emitted from the second light source. As a result, with this culturing device, the life of the haptophytes can be extended as compared with the case of irradiation with the non-yellow visible light alone.

The haptophyte culturing device according to one embodiment may further include a control unit configured to control the first light source and the second light source such that the culture liquid is irradiated with both the yellow light and the visible light. In this case, the haptophytes in the culture liquid are capable of receiving both the yellow light from the first light source and the non-yellow visible light from the second light source, which are emitted under the control of the control unit.

In one embodiment, the haptophytes may be Pavlova lutheri. In this case, the life of Pavlova lutheri can be extended.

### Advantageous Effects of Invention

According to the present disclosure, the life of haptophytes can be extended.

### Brief Description of Drawings

FIG. 1 is an overall perspective view illustrating a culturing device according to an embodiment.
FIG. 2 is a graph showing the absorption spectrum of Pavlova lutheri.
FIG. 3 is a graph showing the time change in Pavlova lutheri cell density at a time of culturing with light different in wavelength range.
FIG. 4 is a graph showing the time changes in Pavlova lutheri cell density according to an example and a comparative example.

### Description of Embodiments

Hereinafter, an embodiment of the present disclosure will be described with reference to the drawings. It should be noted that the same or equivalent elements are denoted by the same reference numerals without redundant description in the following description and drawings. The dimensional ratios in the drawings do not necessarily match those described. The terms of "up", "down", "left", and "right" are based on illustrated states and are for convenience.

### (Haptophytes)

A culturing method according to the present embodiment is a method for efficiently growing haptophytes and extending the life of the grown haptophytes. The haptophytes to be cultured are algae that are a nutritious feed for seedlings of crustaceans such as shrimps and crabs and shellfish such as clams and ark shells. Examples of the haptophytes include Pavlova lutheri, Isochrysis galbana, and Isochrysis sp.

### (Haptophyte Culturing Device)

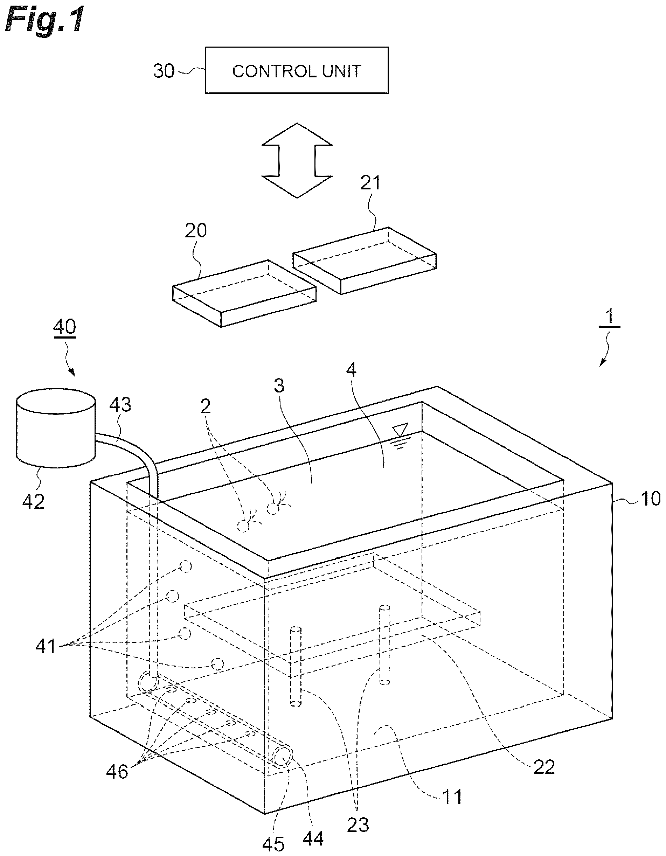

FIG. 1 is an overall perspective view illustrating a culturing device according to the embodiment. The culturing method of the present embodiment is carried out by, for example, a culturing device 1 illustrated in FIG. 1. The culturing device 1 illustrated in FIG. 1 is a device for culturing haptophytes 2. The culturing device 1 includes a culture tank 10, a first illumination 20, a second illumination 21, an underwater illumination 22, a control unit 30, and a ventilation unit 40. The culture tank 10 is a container where the haptophytes 2 and culture liquid 3 are stored. The culture liquid 3 is water suitable for the growing environment of the haptophytes 2 to be cultured. The culture liquid 3 is, for example, water containing more nutrient salt such as nitrogen and phosphorus than seawater. The haptophytes 2 grow by photosynthesis in the culture liquid 3.

A box-shaped space with, for example, an open upper part is defined in the culture tank 10. The haptophytes 2 and the culture liquid 3 are stored in the culture tank 10, and a water surface 4 is formed in the culture tank 10. A bottom surface 11 is provided at the lower end in the culture tank 10.

The first illumination 20 and the second illumination 21 irradiate the haptophytes 2 and the culture liquid 3 in the culture tank 10 with light. The first illumination 20 and the second illumination 21 are disposed above, for example, the water surface 4. The first illumination 20 and the second illumination 21 perform the light irradiation with light-emitting surfaces directed toward the bottom surface 11 of the culture tank 10.

The first illumination 20 irradiates the culture liquid 3 in the culture tank 10 with yellow light. The yellow light is light with a wavelength range of substantially 500 nm or more and 620 nm or less. The wavelength range is the range of the wavelength in which the peak wavelength of the light exists. The upper limit value of the wavelength range of the yellow light may be 600 nm. The lower limit value of the wavelength range of the yellow light may be 540 nm. The lower limit value of the wavelength range of the yellow light may be 550 nm. Here, the yellow light may be, for example, light in which the half-value width of the peak wavelength falls within any of the above wavelength ranges. The first illumination 20 is, for example, a surface light source configured by a plurality of yellow light-emitting diodes (LEDs) fixed on a substrate and having the light-emitting surface on one surface.

The second illumination 21 irradiates the culture liquid 3 in the culture tank 10 with non-yellow visible light. The non-yellow visible light is visible light with a wavelength range of substantially less than 500 nm or more than 620 nm. The non-yellow visible light may be visible light with a wavelength range of more than 600 nm. The non-yellow visible light may be visible light with a wavelength range of less than 540 nm. The non-yellow visible light may be visible light with a wavelength range of less than 550 nm. Here, the non-yellow visible light may be, for example, light in which the half-value width of the peak wavelength falls within any of the above wavelength ranges. The second illumination 21 is, for example, a surface light source configured by a plurality of LEDs fixed on a substrate and having the light-emitting surface on one surface.

The underwater illumination 22 is disposed in the culture tank 10 and irradiates the haptophytes 2 and the culture liquid 3 in the culture tank 10 with light. The underwater illumination 22 is disposed at, for example, the upper end of a scaffold 23 extending upward from the bottom surface 11. The underwater illumination 22 is disposed in the culture liquid 3 away from the water surface 4 and the bottom surface 11. The underwater illumination 22 is disposed along a plane parallel to the bottom surface 11. The underwater illumination 22 is disposed with light-emitting surfaces on both surfaces directed toward the water surface 4 and the bottom surface 11.

The underwater illumination 22 emits yellow light or non-yellow visible light. The wavelength range of the yellow light in the underwater illumination 22 is the same as the wavelength range of the yellow light in the first illumination 20. The wavelength range of the non-yellow visible light in the underwater illumination 22 is the same as the wavelength range of the non-yellow visible light in the second illumination 21. The underwater illumination 22 is, for example, a surface light source configured by laminating the substrates of two LED plates configured by a plurality of LEDs fixed on the substrates and having the light-emitting surfaces on both surfaces.

The underwater illumination 22 is covered with, for example, a translucent resin member. The underwater illumination 22 is waterproofed since the underwater illumination 22 is disposed in the culture liquid 3. A power source is provided in the first illumination 20, the second illumination 21, or the underwater illumination 22. The electric power of the first illumination 20, the second illumination 21, or the underwater illumination 22 may be supplied from an external power source by wiring or the like.

The control unit 30 controls the first illumination 20, the second illumination 21, and the underwater illumination 22 such that the culture liquid 3 is irradiated with both the yellow light and the non-yellow visible light. The control unit 30 is, for example, a central processing unit (CPU). The control unit 30 is connected to the first illumination 20, the second illumination 21, and the underwater illumination 22. The control unit 30 controls the timings at which the first illumination 20, the second illumination 21, and the underwater illumination 22 perform the light irradiation.

The control unit 30 performs control such that, for example, the inside of the culture tank 10 is simultaneously irradiated with the yellow light from the first illumination 20 (an example of a first light source) and the non-yellow visible light from the second illumination 21 (an example of a second light source). The control unit 30 may perform control such that the inside of the culture tank 10 is simultaneously irradiated with the yellow light from the underwater illumination 22 (an example of the first light source) and the non-yellow visible light from the second illumination 21 (an example of the second light source). The control unit 30 may perform control such that the inside of the culture tank 10 is simultaneously irradiated with the yellow light from the first illumination 20 (an example of the first light source) and the non-yellow visible light from the underwater illumination 22 (an example of the second light source).

The control unit 30 may perform control such that the inside of the culture tank 10 is simultaneously irradiated with the yellow light from the combination of the first illumination 20 and the underwater illumination 22 (an example of the first light source) and the non-yellow visible light from the second illumination 21 (an example of the second light source). The control unit 30 may perform control such that the inside of the culture tank 10 is simultaneously irradiated with the yellow light from the first illumination 20 (an example of the first light source) and the non-yellow visible light from the combination of the second illumination 21 and the underwater illumination 22 (an example of the second light source).

The control unit 30 may control the wavelength and intensity of the first illumination 20, the second illumination 21, or the underwater illumination 22. The wavelength and intensity controlled by the control unit 30 are set based on, for example, the density, growth rate, or number of days of culturing of the haptophytes 2 in the culture tank 10. The control unit 30 controls the first illumination 20, the second illumination 21, or the underwater illumination 22 based on the set wavelength and intensity.

The ventilation unit 40 performs ventilation from the bottom surface 11 toward the water surface 4 with a gas 41 containing carbon dioxide and oxygen. The gas 41 contains carbon dioxide and oxygen. The haptophytes 2 perform photosynthesis using the carbon dioxide ventilating the culture liquid 3 and perform respiration using the oxygen. The ventilation unit 40 has an air cylinder 42, a supply pipe 43, and a discharge pipe 44. The air cylinder 42 stores the carbon dioxide- and oxygen-containing gas 41. The air cylinder 42 may be an air pump. One end of the supply pipe 43 is connected to the air cylinder 42, and the other end of the supply pipe 43 is connected to the discharge pipe 44. The supply pipe 43 is made of, for example, a silicon tube. The supply pipe 43 acquires a predetermined amount of the gas 41 from the air cylinder 42. The supply pipe 43 supplies the acquired gas 41 to the discharge pipe 44.

The discharge pipe 44 is disposed on the bottom surface 11. The discharge pipe 44 is a long pipe member. The discharge pipe 44 is made of, for example, a vinyl chloride pipe. A pipe line 45 is defined in the discharge pipe 44, and a plurality of small holes 46 are formed at predetermined intervals in the upper portion of the discharge pipe 44. The plurality of small holes 46 allow the inside of the culture tank 10 and the pipe line 45 to communicate with each other. As for the discharge pipe 44, the gas 41 supplied from the supply pipe 43 to the discharge pipe 44 passes through the pipe line 45 and is discharged into the culture tank 10 from the plurality of small holes 46.

The amount of the gas 41 that the supply pipe 43 acquires from the air cylinder 42, the size of the discharge pipe 44, the shapes and sizes of the cross sections of the pipe line 45 and the small holes 46, and the disposition interval of the small holes 46 in the discharge pipe 44 are appropriately set in accordance with, for example, the density, growth rate, or number of days of culturing of the haptophytes 2. The control unit 30 may control the ventilation unit 40. The control unit 30 controls, for example, the amount of the gas 41 ventilating the culture liquid 3 and the rate at which the gas 41 is supplied. A water flow circulating in the culture tank 10 is generated by the ventilation unit 40 ventilating the culture liquid 3 with the gas 41. As a result, the haptophytes 2 move along the water flow and the inter-individual bias in the amount of light reception is suppressed.

### (Haptophyte Culturing Method)

Next, a method for culturing the haptophytes 2 will be described. The haptophytes 2 and the culture liquid 3 are stored in the culture tank 10. The ventilation unit 40 ventilates the culture liquid 3 with the gas 41 under the control of the control unit 30. The water flow circulating in the culture tank 10 is generated by the ventilation unit 40, and the haptophytes 2 move in the culture tank 10 along the water flow. Under the control of the control unit 30, the haptophytes 2 in the culture liquid 3 stored in the culture tank 10 are irradiated with both the yellow light from the first illumination 20 or the underwater illumination 22 and the non-yellow visible light from the second illumination 21 or the underwater illumination 22. As a result, the haptophytes 2 grow by photosynthesis by receiving both the yellow light and the non-yellow visible light.

### (Summary of Embodiment)

The haptophytes 2 cannot be grown even if the haptophytes 2 are cultured by being irradiated with the yellow light alone. By irradiating the haptophytes 2 with the non-yellow visible light, the haptophytes 2 can be grown, and yet the haptophytes 2 decrease in a short period of time. By culturing the haptophytes 2 with the combination of the non-yellow visible light and the yellow light, which does not contribute to the growth of the haptophytes 2, the haptophytes 2 can be grown and the phenomenon in which the haptophytes 2 decrease in a short period of time is improved. By means of the method for culturing the haptophytes 2 and the culturing device 1 according to the present embodiment, the life of the haptophytes 2 can be extended by irradiating the haptophytes 2 with both the yellow light and the non-yellow visible light.

### (Modification Example)

Although various exemplary embodiments have been described above, various omissions, substitutions, and changes may be made without being limited to the exemplary embodiments described above. For example, the side surface and the bottom surface 11 of the culture tank 10 may have a structure blocking light from the outside such that, for example, light other than the first illumination 20, the second illumination 21, or the underwater illumination 22 does not enter the culture liquid 3.

In addition, the culturing device 1 may lack the first illumination 20. In this case, the underwater illumination 22 is used as a light source for yellow light irradiation. The culturing device 1 may lack the second illumination 21. In this case, the underwater illumination 22 is used as a light source for non-yellow visible light irradiation. The culturing device 1 may lack the first illumination 20 and the second illumination 21. In this case, the underwater illumination 22 emits both the yellow light and the non-yellow visible light. The culturing device 1 may lack the underwater illumination 22. In this case, the first illumination 20 is used as a light source for yellow light irradiation and the second illumination 21 is used as a light source for non-yellow visible light irradiation. The first illumination 20 or the second illumination 21 may be disposed so as to emit light from the side surface or the bottom surface 11 of the culture tank 10.

The culturing device 1 may lack the control unit 30. In this case, a worker may control the light irradiation performed by the first illumination 20, the second illumination 21, or the underwater illumination 22, the amount of the ventilation that the ventilation unit 40 performs with the gas 41, or the supply rate. The culturing method of the present disclosure may be applied to other phytoplankton similar in absorbance characteristics to the haptophytes 2. The phytoplankton are, for example, plankton having low absorbance in the wavelength range of yellow light. As for the culturing method, the irradiation with the yellow light may be performed after the haptophytes have grown by the non-yellow visible light irradiation.

### Examples

Hereinafter, various experiments carried out for verifying the effects of the method and device according to the present disclosure will be described. It should be noted that the present disclosure is not limited to the following experiments. In the following experiments, Pavlova lutheri was used as an example of the haptophytes 2.

### (Absorption Spectrum of Pavlova Lutheri)

The absorption spectrum of Pavlova lutheri was measured by absorptiometry. A device was used in which a light source, opal glass, a sample cell, and a spectroscopic detector are arranged in a straight line in this order. The absorption spectrum of Pavlova lutheri was obtained by storing Pavlova lutheri and water in the sample cell, irradiating the sample cell with scattered light, and detecting the light transmitted through the sample cell for each wavelength. The measurement conditions are as follows.
Sample: Pavlova lutheri and water 1500×10⁴ cells/ml
Light source: Tungsten lamp light source (wavelength range: 350 nm or more and 3000 nm or less (continuous emission))
Optical path length of sample cell: 1 cm
Spectroscopic detector: Multi-channel spectroscopic measuring instrument (PMA) made by Hamamatsu Photonics K.K., linear CCD array method

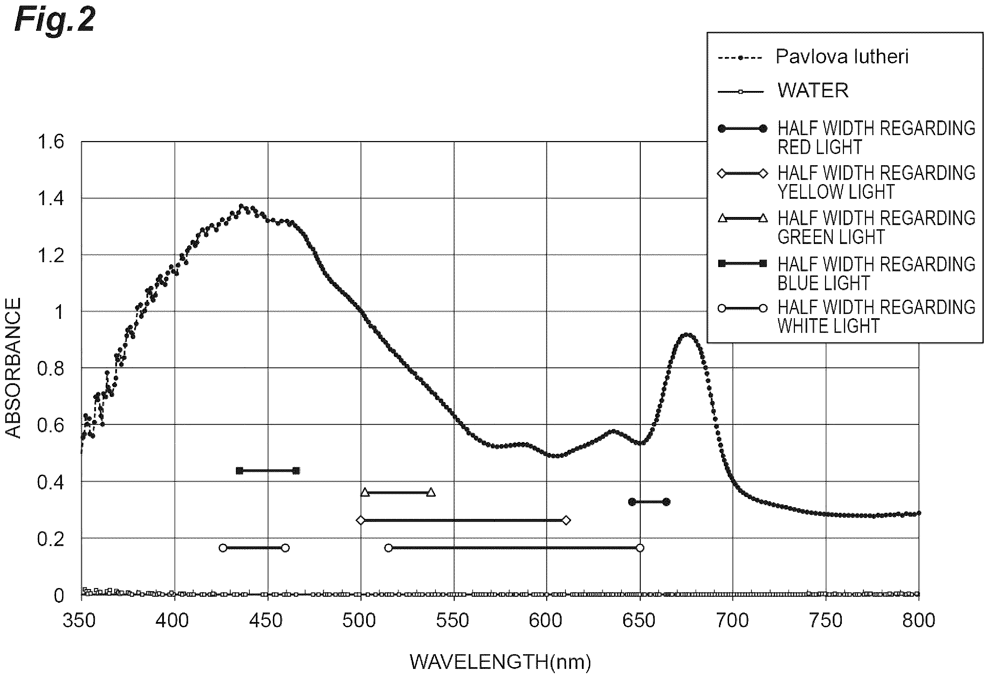

Only water was stored in the sample cell for comparison, and the absorption spectrum of the water was obtained under the above measurement conditions. The results are shown in FIG. 2.

FIG. 2 is a graph showing the absorption spectrum of Pavlova lutheri. The horizontal axis of FIG. 2 is the wavelength (nm), and the vertical axis of FIG. 2 is the absorbance. FIG. 2 shows the half-value widths of the peak wavelengths of the red, yellow, green, blue, and white light emitted from the light source used in the example to be described later. The peak wavelength of the red light is 655 nm, and the half-value width of the red light is in the range of 645 nm or more and 670 nm or less. The peak wavelength of the yellow light is 568 nm, and the half-value width of the yellow light is in the range of 510 nm or more and 620 nm or less. The peak wavelength of the green light is 530 nm, and the half-value width of the green light is in the range of 515 nm or more and 545 nm or less. The peak wavelength of the blue light is 447.5 nm, and the half-value width of the blue light is in the range of 440 nm or more and 460 nm or less. The peak wavelengths of the white light are 440 nm and 570 nm, the half-value width of the short wavelength-side peak is in the range of 430 nm or more and 460 nm or less, and the half-value width of the long wavelength-side peak is in the range of 520 nm or more and 650 nm or less.

As shown in FIG. 2, the absorbance of the water changed near 0 in the wavelength range of 350 nm or more and 800 nm or less. From this, it was confirmed that the effect of the water on the absorption spectrum is small. The absorbance of Pavlova lutheri resulted in a peak around the wavelength range of the blue or red light. In other words, it was confirmed that Pavlova lutheri easily absorbs light around the wavelength range of the blue or red light. This result suggests that the photosynthesis of Pavlova lutheri can be performed more efficiently by using the light around the wavelength range of the blue or red light than by using the yellow or green light.

### (Confirmation of Growth Inhibitory Effect of Yellow Light on Pavlova Lutheri)

The time change in Pavlova lutheri cell density was measured for each irradiation light color. Red, yellow, green, and blue light were used as the irradiation light. The peak wavelengths and half-value widths of the red, yellow, green, and blue light are as described above. The culturing device 1 was prepared for each irradiation light color. The light irradiation was performed using only the underwater illumination 22 without using the first illumination 20 and the second illumination 21. The culture conditions are as follows.
Size (inner dimensions) of culture tank 10: width 309 mm, depth 439 mm, height 300 mm
Water depth of culture liquid 3: 180 mm
Amount of ventilation performed using ventilation unit 40 and gas 41: 8 L/min
Irradiation light intensity: 100 µmolm⁻²s⁻¹ (culture day 0 to 7), 330 µmol/m⁻²s⁻¹ (culture day 8 and later)

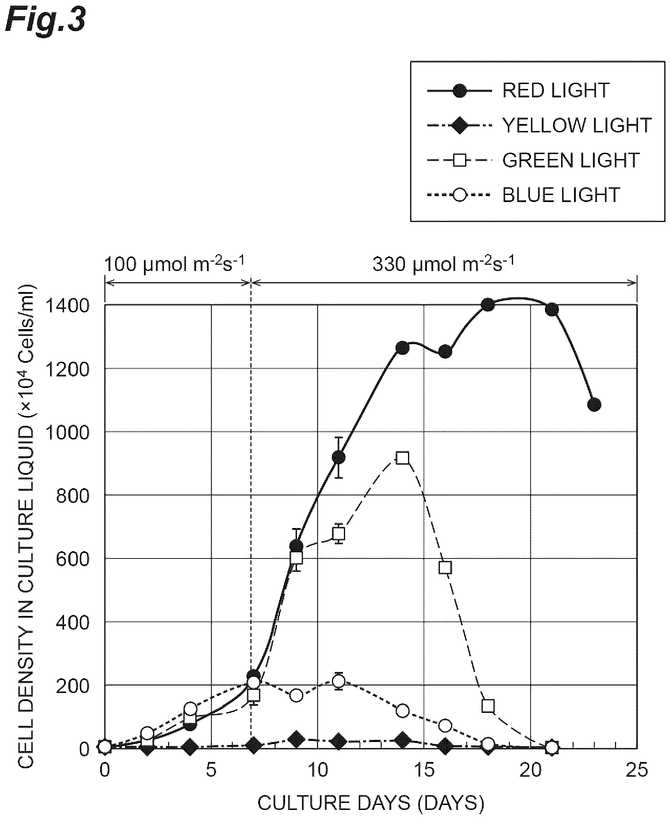

The cell density of Pavlova lutheri cultured under the above culture conditions was measured for each culture day. The cell density of Pavlova lutheri was measured by collecting 10 µL of the culture liquid 3 from the inside of the culture tank 10 and using a one-cell counter (disposable hemocytometer). This measurement was repeated four times, the average was calculated, and the average was used as the cell density of Pavlova lutheri in the culture liquid 3 of that day. The results are shown in FIG. 3.

FIG. 3 is a graph showing the time change in Pavlova lutheri cell density at a time of culturing with light different in wavelength range. The horizontal axis of FIG. 3 is the number of days of culturing (days), and the vertical axis of FIG. 3 is the Pavlova lutheri cell density (×10⁴ cells/ml). The culturing was initiated on day 0. Each graph shown in FIG. 3, which pertains to Pavlova lutheri culturing by means of reception of any one of red, yellow, green, and blue light, plots the Pavlova lutheri cell density for each light and represents an estimated cell density between data points by an approximate line.

As shown in FIG. 3, it was confirmed that the Pavlova lutheri population can be increased the most by the red light among the red, yellow, green, and blue light. As shown in FIG. 2, the red light is easily absorbed by Pavlova lutheri. In other words, it is expected that Pavlova lutheri absorbed a large amount of light and actively photosynthesized by using the red light and the Pavlova lutheri population increased as a result.

However, in the case of culturing using the blue light, which is easily absorbed by Pavlova lutheri, the population did not grow as much as in the case of using the red light and, rather, the population did not grow more than in the case of using the green light, which is lower in absorbance. This result suggests that the absorbance is not the only Pavlova lutheri growth factor. One possible factor is light transmittance. The light transmittance of the culture tank 10 where Pavlova lutheri is present is higher in the order of yellow, red, green, and blue light. Blue light has extremely low transmittance compared to light of other colors. In other words, although blue light is easily absorbed by Pavlova lutheri, the light is hard to reach Pavlova lutheri in a culture tank large in scale as in the present experimental environment, and thus it is presumed that the light contributed less to the population growth than green light.

Further, as shown in FIG. 3, it was confirmed that the yellow light hardly contribute to the growth of Pavlova lutheri despite irradiation. As described above, the yellow light is the light with the highest transmittance although it is not easily absorbed by Pavlova lutheri. Accordingly, it is unlikely that the yellow light did not contribute to the growth of Pavlova lutheri because it did not reach Pavlova lutheri. Microscopic confirmation suggested that Pavlova lutheri was active and photosynthesizing. Then, it can be said that Pavlova lutheri is not growing while performing photosynthesis. In other words, it was confirmed that the yellow light has a unique and extraordinary effect that it does not contribute to growth (suppresses growth) while giving the energy required for the activity of Pavlova lutheri.

Since only the yellow light has a growth inhibitory effect, it can be said that the non-yellow visible light (e.g. red, green, blue, or white light) needs to be emitted for Pavlova lutheri growth. However, it was confirmed that Pavlova lutheri grown by the red, green, or blue light decreases with the passage of culture days. The Pavlova lutheri population reached 0 on day 17 when grown in the blue light and on day 20 when grown in the green light. Even when grown in the red light, the Pavlova lutheri population significantly decreased on day 23 and was expected to reach 0 within a few days. In this manner, it was confirmed that the red, green, and blue light are capable of growing Pavlova lutheri and yet the grown Pavlova lutheri population cannot be maintained for over a week or so and rapidly decreases subsequently.

### (Confirmation of Pavlova Lutheri Life Extension Effect of Yellow Light and Non-Yellow Visible Light)

As an example, Pavlova lutheri was cultured by means of the culturing device 1, the yellow light, and the non-yellow visible light and the time change in cell density was measured. White light was used as an example of the non-yellow visible light. The peak wavelengths and half-value widths of the yellow and white light are as described above. The yellow light was emitted using the underwater illumination 22, and the white light was emitted using the second illumination 21. The culture conditions are as follows.
Size of culture tank 10: width 309 mm, depth 439 mm, height 300 mm
Water depth of culture liquid 3: 180 mm
Amount of ventilation performed using ventilation unit 40 and gas 41: 8 L/min
Intensity of yellow light: 80 µmolm⁻²s⁻¹ (measurement of yellow light from underwater illumination 22 at 60 mm above underwater illumination 22)
Intensity of white light: 200 µmolm⁻²s⁻¹ (measurement of white light from second illumination 21 on water surface)

The cell density of Pavlova lutheri cultured under the above culture conditions was measured by the method described above for each culture day.

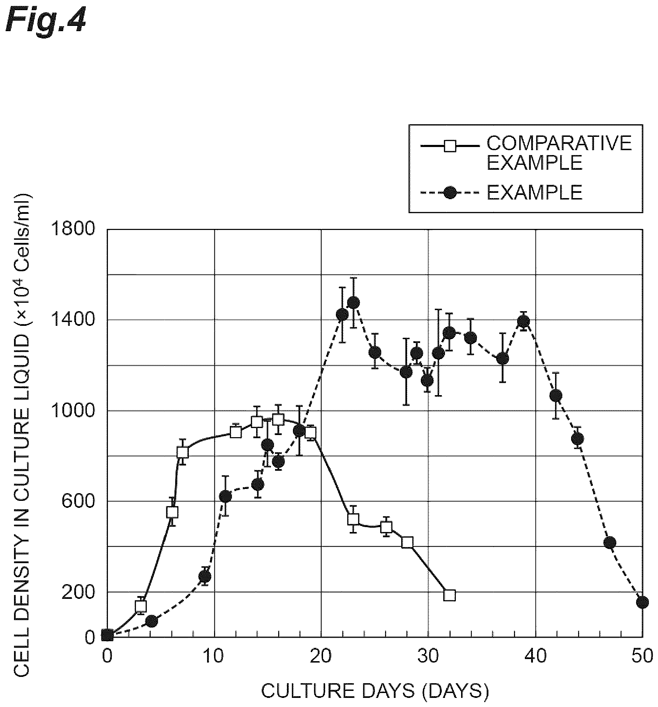

As a comparative example, Pavlova lutheri was cultured only with white light and using another culturing device 1 and the time change in cell density was measured. The white light was emitted using the second illumination 21. The culture conditions and the cell density measurement method were the same as those of the example with the exception of the yellow light not emitted in the comparative example. The results are shown in FIG. 4.

FIG. 4 is a graph showing the time changes in Pavlova lutheri cell density according to the example and comparative example. The horizontal axis of FIG. 3 is the number of days of culturing (days), and the vertical axis of FIG. 3 is the Pavlova lutheri cell density (×10⁴ cells/ml). The culturing was initiated on day 0. Each graph shown in FIG. 4 plots the Pavlova lutheri cell density according to the example or comparative example and represents an estimated cell density between data points by an approximate line.

As shown in FIG. 4, the Pavlova lutheri cell density according to the comparative example decreased with the passage of culture days from day 7 although the density increases in a short period of time from the culturing initiation as in the case of the non-yellow visible light shown in FIG. 3. Specifically, the increase in cell density slowed down from day 7 and the cell density was maintained at 800 to 1000 (×10⁴ cells/ml) from day 7 to day 19. The cell density decreased after day 19. The Pavlova lutheri population was 800 (×10⁴ cells/ml) or more for 12 days.

In contrast, it was confirmed that Pavlova lutheri according to the example grows with stability although the growth rate from the culturing initiation is slightly lower than that of the comparative example. Specifically, the cell density increased until day 22. The increase in cell density slowed down from day 22, and the cell density was maintained at 1100 to 1500 (×10⁴ cells/ml) from day 22 to day 39. The cell density decreased after day 39. The Pavlova lutheri population was 800 (×10⁴ cells/ml) or more for 28 days.

As described above, in the example, it was confirmed that the period from the initiation of culturing by light irradiation to a rapid decrease in population is 39 days, which is 20 days longer than in the comparative example. In the example, it was confirmed that the maximum value of the cell density was maintained for 17 days, which is five days longer than in the comparative example. In the examples, it was confirmed that 800 (×10⁴ cells/ml) or more was maintained for 28 days, which is 16 days longer than in the comparative example. In this manner, it was confirmed that the culturing method according to the example is longer in every evaluation period than the method according to the comparative example. Accordingly, it was confirmed that the culturing method according to the example enables life extension for haptophytes such as Pavlova lutheri as compared with the method according to the comparative example.

Further, in the example, the maximum value of the cell density was 1100 to 1500 (×10⁴ cells/ml), which is higher than 800 to 1000 (×10⁴ cells/ml) of the comparative example. In this manner, it was confirmed that the maximum value of the Pavlova lutheri cell density can be increased by the culturing method according to the example as compared with the method according to the comparative example.

### Reference Signs List

1: culturing device, 2: haptophytes, 3: culture liquid, 4: water surface, 10: culture tank, 11: bottom surface, 20: first illumination, 21: second illumination, 22: underwater illumination, 23: scaffold, 30: control unit, 40: ventilation unit, 41: gas, 42: air cylinder, 43: supply pipe, 44: discharge pipe, 45: pipe line, 46: small hole.

## Claims

1. A method for culturing haptophytes comprising simultaneously irradiating culture liquid containing the haptophytes with both
- yellow light the half-value width of the peak wavelength of which falls within the wavelength range of 550-600 nm, using a first light source, and
- non-yellow visible light the peak wavelength of which falls within the wavelength range of < 550 nm or within the wavelength range of > 600 nm, using a second light source.

2. The method of claim 1, wherein the haptophytes are Pavlova lutheri.

3. A device for culturing haptophytes comprising:
- a culture tank storing culture liquid containing the haptophytes;
- a first light source for irradiating the culture liquid in the culture tank with yellow light the half-value width of the peak wavelength of which falls within the wavelength range of 550-600 nm;
- a second light source irradiating the culture liquid in the culture tank with non-yellow visible light the peak wavelength of which falls within the wavelength range of < 550 nm or within the wavelength range of > 600 nm, and
- a control unit configured to control the first light source and the second light source such that the culture liquid is irradiated with both the yellow light and the visible light.

4. The device of claim 3, wherein the haptophytes are Pavlova lutheri.

## Patentansprüche

1. Verfahren zur Kultivierung von Haptophyten, umfassend die gleichzeitige Bestrahlung einer Kulturflüssigkeit, die die Haptophyten enthält, mit sowohl
- gelbem Licht, dessen Halbwertsbreite der Peak-Wellenlänge in den Wellenlängenbereich von 550-600 nm fällt, unter Verwendung einer ersten Lichtquelle, und
- nicht-gelbem sichtbarem Licht, dessen Peak-Wellenlänge in den Wellenlängenbereich von < 550 nm oder in den Wellenlängenbereich von > 600 nm fällt, unter Verwendung einer zweiten Lichtquelle.

2. Verfahren nach Anspruch 1, bei dem die Haptophyten Pavlova lutheri sind.

3. Vorrichtung zur Kultivierung von Haptophyten, umfassend:
- einen Kulturtank, in dem eine Kulturflüssigkeit , die die Haptophyten enthält, aufbewahrt wird;
- eine erste Lichtquelle zur Bestrahlung der Kulturflüssigkeit in dem Kulturtank mit gelbem Licht, dessen Halbwertsbreite der Peak-Wellenlänge in den Wellenlängenbereich von 550-600 nm fällt;
- eine zweite Lichtquelle zur Bestrahlung der Kulturflüssigkeit in dem Kulturtank mit nicht-gelbem sichtbarem Licht, dessen Peak-Wellenlänge in den Wellenlängenbereich von < 550 nm oder in den Wellenlängenbereich von > 600 nm fällt, und
- eine Steuereinheit, die so konfiguriert ist, dass sie die erste Lichtquelle und die zweite Lichtquelle so steuert, dass die Kulturflüssigkeit sowohl mit dem gelben Licht als auch mit dem sichtbaren Licht bestrahlt wird.

4. Die Vorrichtung nach Anspruch 3, wobei die Haptophyten Pavlova lutheri sind.

## Revendications

1. Une méthode de culture des haptophytes comprenant l'irradiation simultanée d'un liquide de culture contenant les haptophytes avec à la fois
- une lumière jaune dont la largeur de la demi-valeur de la longueur d'onde de crête se situe dans le domaine de longueurs d'onde de 550-600 nm, en utilisant une première source lumineuse, et
- une lumière visible non jaune dont la longueur d'onde de crête se situe dans le domaine de longueurs d'onde < 550 nm ou dans le domaine de longueurs d'onde > 600 nm, en utilisant une deuxième source lumineuse.

2. La méthode de la revendication 1, dans laquelle les haptophytes sont des Pavlova lutheri.

3. Un dispositif de culture d'haptophytes comprenant :
- un réservoir de culture stockant le liquide de culture contenant les haptophytes ;
- une première source de lumière pour irradier le liquide de culture dans le réservoir de culture avec de la lumière jaune dont la largeur de la demi-valeur de la longueur d'onde de crête se situe dans le domaine de longueurs d'onde de 550-600 nm ;
- une deuxième source lumineuse irradiant le liquide de culture dans le réservoir de culture avec une lumière visible non jaune dont la longueur d'onde de crête se situe dans le domaine de longueurs d'onde < 550 nm ou dans le domaine de longueurs d'onde > 600 nm, et
- une unité de contrôle configurée pour contrôler la première source de lumière et la deuxième source de lumière de manière à ce que le liquide de culture soit irradié à la fois par la lumière jaune et par la lumière visible.

4. Le dispositif de la revendication 3, dans lequel les haptophytes sont des Pavlova lutheri.
